(19) **Europäisches Patentamt**

**European Patent Office**

**Office européen des brevets**

(11) **EP 1 219 292 B1**

(12) **EUROPÄISCHE PATENTSCHRIFT**

(45) Veröffentlichungstag und Bekanntmachung des Hinweises auf die Patenterteilung:
**06.10.2004 Patentblatt 2004/41**

(51) Int Cl.⁷: **A61K 9/14**, A61K 31/07

(21) Anmeldenummer: **01128813.1**

(22) Anmeldetag: **04.12.2001**

(54) **Verfahren zur Herstellung von Trockenpulvern eines oder mehrerer Sauerstoff-haltiger Carotinoide**

Procedure or manufacturing dry powdered compositions comprising one or several oxygen-containing carotenoids

Procédure de préparation d'une poudre sèche d'un ou de plusieurs carotenoides contenant de l'oxygène

(84) Benannte Vertragsstaaten:
**AT BE CH CY DE DK ES FI FR GB GR IE IT LI LU MC NL PT SE TR**
Benannte Erstreckungsstaaten:
**RO SI**

(30) Priorität: **21.12.2000 DE 10064387**

(43) Veröffentlichungstag der Anmeldung:
**03.07.2002 Patentblatt 2002/27**

(73) Patentinhaber: **BASF AKTIENGESELLSCHAFT**
**67056 Ludwigshafen (DE)**

(72) Erfinder:
• **Runge, Frank, Dr.**
**67159 Friedelsheim (DE)**

• **Lüddecke, Erik, Dr.**
**67112 Mutterstadt (DE)**
• **Auweter, Helmut, Dr.**
**67117 Limburgerhof (DE)**
• **Pfeiffer, Angelika-Maria, Dr.**
**67134 Birkenheide (DE)**
• **Hinz, Willy, Dr.**
**68199 Mannheim (DE)**

(56) Entgegenhaltungen:
EP-A- 0 547 422        EP-A- 0 807 431
WO-A-91/06292          WO-A-96/08977
DE-A- 19 652 287       DE-A- 19 841 930
US-A- 4 522 743

**Beschreibung**

Die Erfindung betrifft ein Verfahren zur Herstellung von Trockenpulvern eines oder mehrerer Sauerstoff-haltiger Carotinoide, insbesondere von Trockenpulvern, enthaltend Carotinoide, ausgewählt aus der Gruppe, bestehend aus Astaxanthin, Canthaxanthin, Lutein, Zeaxanthin, Citranaxanthin und β-Apo-8'-Carotinsäureethylester.

Die Stoffklasse der Carotinoide klassifiziert man in zwei Hauptgruppen, die Carotine und die Xanthophylle. Im Unterschied zu den Carotinen, bei denen es sich um reine Polyen-Kohlenwasserstoffe handelt, wie beispielsweise β-Carotin oder Lycopin, kommen in den Xanthophyllen noch Sauerstoff-Funktionen wie Hydroxy-, Epoxy- und/oder Oxogruppen vor. Typische Vertreter dieser Gruppe sind u.a. Astaxanthin, Canthaxanthin, Lutein und Zeaxanthin.

Sauerstoff-haltige Carotinoide sind in der Natur weit verbreitet und kommen u.a. im Mais (Zeaxanthin), in grünen Bohnen (Lutein), in Paprika (Capsanthin), in Eidottern (Lutein) sowie in Krebsen und Lachsen (Astaxanthin) vor, wobei sie diesen Nahrungsmitteln ihre charakteristische Färbung verleihen.

Diese sowohl synthetisch zugänglichen als auch aus natürlichen Quellen isolierbaren Polyene stellen für die Lebens- und Futtermittelindustrie und für den pharmazeutischen Bereich wichtige Farbkörper dar und sind, wie im Falle von Astaxanthin, Wirkstoffe mit Provitamin-A Aktivität beim Lachs.

Xanthophylle sind wie alle Carotinoide in Wasser unlöslich, während in Fetten und Ölen eine jedoch nur geringe Löslichkeit gefunden wird. Diese begrenzte Löslichkeit sowie die hohe Oxidationsempfindlichkeit stehen einer direkten Anwendung der relativ grobkörnigen bei der Synthese erhaltenen Produkte in der Einfärbung von Lebens- und Futtermitteln entgegen, da die Substanzen in grobkristalliner Form nur schlechte Färbungsergebnisse liefern. Diese für die praktische Verwendung der Xanthophylle nachteiligen Effekte wirken sich insbesondere im wäßrigen Medium aus.

Nur durch gezielt hergestellte Formulierungen, in denen die Wirkstoffe in fein verteilter Form und gegebenenfalls durch Schutzkolloide oxidationsgeschützt vorliegen, lassen sich bei der direkten Einfärbung von Lebensmitteln verbesserte Farbausbeuten erzielen. Außerdem führen diese in Futtermitteln verwendeten Formulierungen zu einer höheren Bioverfügbarkeit der Xanthophylle und damit indirekt zu besseren Färbungseffekten z.B. bei der Eidotter- oder Fischpigmentierung.

Zur Verbesserung der Farbausbeuten und zur Erhöhung der Resorbierbarkeit bzw. Bioverfügbarkeit sind verschiedene Verfahren beschrieben worden, die alle das Ziel haben, die Kristallitgröße der Wirkstoffe zu verkleinern und auf einen Teilchengrößenbereich von kleiner 10 μm zu bringen.

Zahlreiche Methoden, u.a. beschrieben in Chimia 21, 329 (1967), WO 91/06292 sowie in WO 94/19411, bedienen sich dabei der Vermahlung von Carotinoiden mittels einer Kolloidmühle und erzielen damit Partikelgrößen von 2 bis 10 μm.

Daneben existieren eine Reihe von kombinierten Emulgier-/Sprühtrocknungsverfahren, wie sie z.B. in DE-A-12 11 911 oder in EP-A-0 410 236 beschrieben sind.

Gemäß der europäischen Patentschrift EP-B-0 065 193 erfolgt die Herstellung von feinverteilten, pulverförmigen Carotinoidpräparaten dadurch, daß man ein Carotinoid in einem flüchtigen, mit Wasser mischbaren organischen Lösungsmittel bei erhöhten Temperaturen, gegebenenfalls unter erhöhtem Druck löst, das Carotinoid durch Mischen mit einer wäßrigen Lösung eines Schutzkolloids ausfällt und anschließend sprühtrocknet.

Ein analoges Verfahren zur Herstellung von feinverteilten, pulverförmigen Carotinoidpräparaten wird in EP-A-0 937 412 unter Verwendung von mit Wasser nicht mischbaren Lösungsmitteln beschrieben.

Bei den nach EP-B-0 065 193 hergestellten nanopartikulären Wirkstoffdispersionen von Xanthophyllen sind jedoch häufig folgende Phänomene zu beobachten.

Die wäßrigen, Xanthophyll-haltigen Wirkstoffdispersionen sind häufig, insbesondere bei der Aufkonzentration, kolloidal instabil. Durch Ausflockungen der Wirkstoffpartikel, die dabei teilweise sedimentieren, teilweise aufrahmen, ist eine weitere Überführung der Dispersion in ein Trockenpulver nicht mehr möglich.

Bei Xanthophyllen mit Carbonyl-Funktionen kann außerdem die als Schutzkolloid eingesetzte Gelatine vernetzen, so daß ein Gel entsteht, das nicht mehr redispergierbar ist und das ebenfalls nicht weiter in ein Trockenpulver überführt werden kann.

Somit können die hohen Anforderungen an Xanthophyll-haltigen Formulierungen bezüglich Farbwirkung und Bioverfügbarkeit aufgrund der geschilderten Problematik mit dem o.g. Verfahren nicht immer erfüllt werden.

Nachteilig an Gelatinen sind auch deren stark klebenden Eigenschaften. Mit den für flüssige Systeme üblichen Trocknungsmethoden wie die Sprühtrocknung oder Sprühwirbelbett-Trocknung kann es bei Verwendung von gelatinehaltigen Produkten zu Fadenbildung oder Verbackungen kommen.

Hinzu kommt eine immer geringer werdende Akzeptanz des Verbrauchers gegenüber Gelatine-haltigen Produkten.

In andere oft verwendete Schutzkolloide wie Gummi arabicum, Stärke, Dextrine, Pektin oder Tragant lassen sich häufig nur relativ geringe Konzentrationen von fettlöslichen Substanzen einbetten. Darüber hinaus stand insbesondere Gummi arabicum in der Vergangenheit infolge von Mißernten nicht immer und in ausreichender Qualität zur Verfügung.

Synthetische Kolloide wie Polyvinylpyrrolidon oder partialsynthetische Polymere wie Cellulosederivate zeigen ebenfalls eine begrenzte Emulgierkapazität und werden vor allem im Lebensmittelbereich nicht immer akzeptiert.

DE-A-44 24 085 beschreibt die Verwendung von teilabgebauten Sojaproteinen als Schutzkolloide für fettlösliche Wirkstoffe. Die hier offenbarten Sojaproteine weisen einen Abbaugrad von 0,1 bis 5 % auf.

Nachteilig an den oben genannten Sojaproteinen sind häufig deren schlechte Wasserlöslichkeit, unzureichende Emulgiereigenschaften sowie deren Tendenz zur Vernetzung, die besonders für die Herstellung von in Wasser redispergierbarer Trockenpulver unerwünscht ist.

Aufgabe der vorliegenden Erfindung war es daher, Verfahren zur Herstellung von Trockenpulvern Sauerstoffhaltiger Carotinoide unter Verwendung von Schutzkolloiden vorzuschlagen, die die oben genannten Nachteile des Standes der Technik nicht aufweisen.

Diese Aufgabe wurde erfindungsgemäß gelöst mit einem Verfahren zur Herstellung von Trockenpulvern eines oder mehrerer Sauerstoff-haltiger Carotinoide durch

a) Dispergieren eines oder mehrerer Sauerstoff-haltiger Carotinoide in einer wäßrigen molekulardispersen oder kolloiddispersen Lösung eines Schutzkolloids und

b) Überführung der gebildeten Dispersion in ein Trockenpulver durch Abtrennung des Wassers und gegebenenfalls zusätzlich verwendeter Lösungsmittel und Trocknung, gegebenenfalls in Gegenwart eines Überzugsmaterials,

dadurch gekennzeichnet, daß man als Schutzkolloid im Verfahrensschritt a) mindestens ein teilabgebautes Sojaprotein mit einem Abbaugrad von größer 5 % verwendet.

Erfindungsgemäß werden als Schutzkolloide teilabgebaute Sojaproteine verwendet, welche einen Abbaugrad ("DH": "degree of hydrolysis") von größer 5 %, bevorzugt 6 bis 20 %, besonders bevorzugt 6 bis 12 %, ganz besonders bevorzugt 6 bis 9 % aufweisen. Der Abbaugrad "DH" ist folgendermaßen definiert:

$$DH = \frac{\text{Anzahl der gespaltenen Peptidbindungen}}{\text{Gesamtzahl der Peptidbindungen}} \times 100\,\%$$

Der Abbaugrad kann gemäß der sogenannten "pH-Stat-Methode" bestimmt werden, wie von C.F. Jacobsen et al. in "Methods of Biochemical Analysis", Vol. IV, S. 171-210, Interscience Publishers Inc., New York 1957, beschrieben.

Der Teilabbau erfolgt in der Regel enzymatisch, wobei als geeignete Enzyme Proteasen aus Pflanzen, Mikroorganismen, Pilzen oder tierische Proteasen in Betracht kommen. Vorzugsweise erfolgt der Teilabbau mit der pflanzlichen Protease Bromelain.

Als Sojaproteine werden üblicherweise handelsübliche Sojaprotein-Isolate und -Konzentrate mit Proteingehalten von 70 bis 90 Gew.-% eingesetzt, wobei die restlichen 10 bis 30 Gew.-% mehr oder weniger undefinierte andere Pflanzenbestandteile darstellen. Als bevorzugt verwendete Sojaproteine seien in diesem Zusammenhang nicht genmodifizierte Sojaproteine genannt.

Die Sojaprotein-Isolate werden in wäßrigem Medium mit dem Enzym inkubiert, vorzugsweise bei Temperaturen von 50 bis 70°C und pH-Werten von 7 bis 9. Das geeignete Verhältnis Protein zu Enzym kann im Einzelfall für den gewünschten Abbaugrad in für den Fachmann einfachen Laborversuchen ermittelt werden.

Die wäßrigen Sojaproteinhydrolysat-Lösungen werden in der Regel so hergestellt, daß der Proteingehalt 6 bis 10 Gew.-% beträgt.

Das gewichtsdurchschnittliche Molekulargewicht der erfindungsgemäß verwendeten, teilabgebauten Sojaproteine liegt im Bereich von 15000 bis 250000, bevorzugt von 25000 bis 220000, besonders bevorzugt von 50000 bis 200000, ganz besonders bevorzugt im Bereich von 120000 bis 180000.

Es ist auch möglich, in dem erfindungsgemäßen Verfahren Mischungen von teilabgebauten Sojaproteinen unterschiedlicher Abbaugrade oder Mischungen aus teilabgebauten und nichtabgebauten Sojaproteinen als Schutzkolloide zu verwenden. Bei diesen Mischungen liegen deren gewichtsdurchschnittliche Molekulargewichte ebenfalls in den oben genannten Bereichen.

Unter dem Begriff Dispergieren ist bevorzugt die Herstellung wäßriger Suspensionen sowie wäßriger Emulsionen zu verstehen. Besonders bevorzugt handelt es sich beim Dispergierschritt a) um die Herstellung einer Suspension eines oder mehrerer Sauerstoff-haltiger Carotinoide in einer wäßrigen molekulardispersen oder kolloiddispersen Lösung eines teilabgebauten Sojaproteins, bei der die dispergierte Phase mindestens einen der Wirkstoffe als nanopartikuläre Teilchen enthält.

Eine bevorzugte Ausführungsform des o.g. Verfahrens ist dadurch gekennzeichnet, daß man die im Verfahrensschritt a) hergestellte Suspension vor der Überführung in ein Trockenpulver mahlt. In diesem Fall wird der Wirkstoff vor dem Mahlvorgang bevorzugt in kristalliner Form suspendiert.

Die Mahlung kann dabei in an sich bekannter Weise z.B. mit einer Kugelmühle erfolgen. Dabei wird je nach verwendetem Mühlentyp so lange gemahlen, bis die Teilchen eine über Fraunhofer Beugung ermittelte mittlere Partikelgröße D[4,3] von 0,1 bis 100 µm, bevorzugt 0,2 bis 50 µm, besonders bevorzugt 0,2 bis 20 µm, ganz besonders bevorzugt 0,2 bis 5 µm, insbesondere 0,2 bis 0,8 µm aufweisen. Der Begriff D[4,3] bezeichnet den volumengewichteten mittleren Durchmesser (siehe Handbuch zu Malvern Mastersizer S, Malvern Instruments Ltd., UK).

Nähere Einzelheiten zur Mahlung und den dafür verwendeten Apparaturen finden sich u.a. in Ullmann's Encyclopedia of Industrial Chemistry, Sixth Edition, 1999, Electronic Release, Size Reduction, Kapitel 3.6.: Wet Grinding sowie in EP-A-0 498 824.

Eine ebenfalls bevorzugte Variante des erfindungsgemäßen Verfahrens ist dadurch gekennzeichnet, daß das Dispergieren in der Stufe a) folgende Schritte enthält:

$a_1$) Lösen eines oder mehrerer Sauerstoff-haltiger Carotinoide in einem mit Wasser mischbaren, organischen Lösungsmittel oder in einer Mischung aus Wasser und einem mit Wasser mischbaren, organischen Lösungsmittel oder

$a_2$) Lösen eines oder mehrerer Sauerstoff-haltiger Carotinoide in einem mit Wasser nicht mischbaren, organischen Lösungsmittel und

$a_3$) Mischen der nach $a_1$) oder $a_2$) erhaltenen Lösung mit einer wäßrigen molekulardispersen oder kolloiddispersen Lösung eines teilabgebauten Sojaproteins mit einem Abbaugrad von größer 5 %, wobei die hydrophobe Phase des Carotinoids als nanodisperse Phase entsteht.

Je nach Art der verwendeten Lösungsmittel kann es sich bei der nanodispersen Phase im Schritt $a_3$) um feste Nanopartikel (Suspension) oder um Nanotröpfchen (Emulsion) handeln.

Die in der Stufe $a_1$) verwendeten wassermischbaren Lösungsmittel sind vor allem wassermischbare, thermisch stabile, flüchtige, nur Kohlenstoff, Wasserstoff und Sauerstoff enthaltenene Lösungsmittel wie Alkohole, Ether, Ester, Ketone und Acetale zu nennen. Zweckmäßig verwendet man solche Lösungsmittel, die mindestens zu 10 % wassermischbar sind, einen Siedepunkt unter 200°C aufweisen und/oder weniger als 10 Kohlenstoffe haben. Besonders bevorzugt werden Methanol, Ethanol, n-Propanol, Isopropanol, 1,2-Butandiol-1-methylether, 1,2-Propandiol-1-n-propylether, Tetrahydrofuran oder Aceton verwendet.

Der Begriff "ein mit Wasser nicht mischbares organisches Lösungsmittel" steht im Sinne der vorliegenden Erfindung für ein organisches Lösungsmittel mit einer Wasserlöslichkeit bei Normaldruck von weniger als 10 %. Als mögliche Lösungsmittel kommen dabei u.a. halogenierte aliphatische Kohlenwasserstoffe, wie z.B. Methylenchlorid, Chloroform und Tetrachlorkohlenstoff, Carbonsäureester wie Dimethylcarbonat, Diethylcarbonat, Propylencarbonat, Ethylformiat, Methyl-, Ethyl- oder Isopropylacetat sowie Ether wie Methyl-tert. butylether in Frage. Bevorzugte, mit Wasser nicht mischbare organische Lösungsmittel sind die folgenden Verbindungen aus der Gruppe, bestehend aus Dimethylcarbonat, Propylencarbonat, Ethylformiat, Ethylacetat, Isopropylacetat und Methyl-tert. butylether.

Bei dem erfindungsgemäßen Verfahren handelt es sich bevorzugt um die Herstellung von Trockenpulvern von Sauerstoff-haltigen Carotinoiden, ausgewählt aus der Gruppe, bestehend Astaxanthin, Canthaxanthin, Lutein, Zeaxanthin, Citranaxanthin und β-Apo-8'-Carotinsäureethylester handelt.

Besonders bevorzugt ist das erfindungsgemäße Verfahren dadurch gekennzeichnet ist, daß man

a) Astaxanthin und/oder Canthaxanthin in einem mit Wasser mischbaren, organischen Lösungsmittel oder einer Mischung aus Wasser und einem mit Wasser mischbaren, organischen Lösungsmittel bei Temperaturen größer 30°C löst,

b) die erhaltene Lösung mit einer wäßrigen molekulardispersen oder kolloiddispersen Lösung eines teilabgebauten Sojaproteins mit einem Abbaugrad von 6 bis 9 % mischt und

c) die gebildete Dispersion in ein Trockenpulver überführt.

Ganz besonders bevorzugt handelt es sich hierbei um ein Verfahren zur Herstellung von Astaxanthin-haltigen Trockenpulvern.

Die Herstellung der o.g. Trockenpulver erfolgt vorteilhafterweise so, daß man mindestens eines der Sauerstoff-haltigen Carotinoide in einem mit Wasser mischbaren, organischen Lösungsmittel bei Temperaturen größer 30°C, vorzugsweise zwischen 50°C und 240°C, insbesondere 100°C bis 200°C, besonders bevorzugt 140°C bis 180°C, gegebenenfalls unter Druck, löst.

Da die Einwirkung hoher Temperaturen u.U. den gewünschten hohen all-trans Isomerenanteil herabsetzen kann, löst man das/die Carotinoid(e) möglichst rasch, beispielsweise im Sekundenbereich, z.B. in 0,1 bis 10 Sekunden, besonders bevorzugt in weniger als 1 Sekunde. Zur raschen Herstellung der moleculardispersen Lösung kann die Anwendung von erhöhtem Druck, z.B. im Bereich von 20 bar bis 80 bar, vorzugsweise 30 bis 60 bar, vorteilhaft sein.

Die so erhaltene molekulardisperse Lösung versetzt man anschließend direkt mit der gegebenenfalls gekühlten wäßrigen molekulardispersen oder kolloiddispersen Lösung des Schutzkolloids in der Weise, daß sich eine Mischungstemperatur von etwa 35°C bis 80°C einstellt.

Dabei wird die Lösungsmittelkomponente in die wäßrige Phase überführt und die hydrophobe Phase des/der Carotinoid(e) entsteht als nanodisperse Phase.

Hinsichtlich einer näheren Verfahrens- und Apparatebeschreibung zur oben genannten Dispergierung wird an dieser Stelle auf EP B-0 065 193 Bezug genommen.

Die Überführung in ein Trockenpulver kann dabei u.a. durch Sprühtrocknung, Sprühkühlung, Gefriertrocknung oder Trocknung im Wirbelbett, gegebenenfalls auch in Gegenwart eines Überzugsmaterials erfolgen. Als Überzugsmittel eignen sich u.a. Maisstärke, Kieselsäure oder auch Tricalciumphosphat.

Zur Erhöhung der mechanischen Stabilität des Endproduktes kann es in einigen Fällen zweckmäßig sein, dem Kolloid einen Weichmacher zuzusetzen, wie Zucker oder Zuckeralkohole, z.B. Saccharose, Glucose, Glucosesirup, Dextrin, Lactose, Invertzucker, Sorbit, Mannit oder Glycerin.

Zur Erhöhung der Stabilität des Wirkstoffes gegen oxidativen Abbau ist es vorteilhaft, Stabilisatoren wie $\alpha$-Tocopherol, t-Butylhydroxy-toluol, t-Butylhydroxyanisol, Ascorbinsäure oder Ethoxyquin zuzusetzen. Sie können entweder der wäßrigen oder der Lösungsmittel-Phase zugesetzt werden, vorzugsweise werden sie jedoch gemeinsam mit den Wirkstoffen in der Lösungsmittel-Phase gelöst.

Unter Umständen kann es auch vorteilhaft sein, zusätzlich in der Lösungsmittel-Phase ein physiologisch zugelassenes Öl wie beispielsweise Sesamöl, Maiskeimöl, Baumwollsaatöl, Sojabohnenöl oder Erdnußöl sowie Ester mittelkettiger pflanzlicher Fettsäuren in einer Konzentration von 0 bis 500 Gew.-%, vorzugsweise 10 bis 300 Gew.-%, besonders bevorzugt 20 bis 100 Gew.-%, bezogen auf das/die Xanthophyll(e), zu lösen, das dann gemeinsam mit den Wirkstoffen und den genannten Zusatzstoffen beim Mischen mit der wäßrigen Phase extrem feinteilig ausgefällt wird.

Das Verhältnis Schutzkolloid und Weichmacher zu Sauerstoff-haltigem Carotinoid wird im allgemeinen so gewählt, daß ein Endprodukt erhalten wird, das zwischen 0,1 und 30 Gew.-%, bevorzugt 1 bis 25 Gew.-%, besonders bevorzugt 5 bis 20 Gew.-% Carotinoid, 10 bis 70 Gew.-% eines Schutzkolloids, 10 bis 70 Gew.-% eines Weichmachers, alle Prozentangaben bezogen auf die Trockenmasse des Pulvers, sowie gegebenenfalls geringe Mengen eines Stabilisators enthält.

Die Erfindung betrifft auch Trockenpulver von Sauerstoff-haltigen Carotinoiden, erhältlich nach einem der eingangs genannten Verfahren.

Bevorzugt handelt es sich dabei um Trockenpulver, enthaltend Sauerstoff-haltige Carotinoide, ausgewählt aus der Gruppe, bestehend Astaxanthin, Canthaxanthin, Lutein, Zeaxanthin, Citranaxanthin und $\beta$-Apo-8'-Carotinsäureethylester, besonders bevorzugt Canthaxanthin und Astaxanthin, ganz besonders bevorzugt Astaxanthin.

Der Wirkstoffgehalt in den erfindungsgemäßen Trockenpulvern liegt im Bereich von 0,1 bis 30 Gew.-%, bevorzugt 1 bis 25 Gew.-%, besonders bevorzugt 5 bis 20 Gew.-%, ganz besonders bevorzugt im Bereich von 8 bis 15 Gew.-%.

Die erfindungsgemäßen Trockenpulver zeichnen sich u.a. dadurch aus, daß sie sich in wäßrigen Systemen unter Erzielung einer gleichmäßigen Feinverteilung des Wirkstoffes im Korngrößenbereich kleiner 1 $\mu$m problemlos wieder redispergieren lassen.

Darüberhinaus wurde gefunden, daß man kolloidal stabile und nicht vernetzende nanopartikuläre Wirkstoffdispersionen von Sauerstoff-haltigen Carotinoiden erhält, deren Viskositätsverhalten annähernd denen von Newtonschen Flüssigkeiten entspricht. Derartige Flüssigkeiten zeichnen sich dadurch aus, daß deren durch die Newtonsche Gleichung $\tau = h \cdot D$ definierter Fließwiderstand bei gegebener Temperatur eine Stoffkonstante ist ($\tau$ = Schubspannung, D = Schergefälle, h = dynamische Viskosität). Die graphische Darstellung des Fließverhaltens newtonscher Flüssigkeiten ergibt bei gegebener Temperatur annähernd eine Gerade. Insbesondere ändert sich die Viskosität der Wirkstoffdispersion bei 40°C und bei 60°C im Scherbereich zwischen $10^{-2}$ sec$^{-1}$ und $10^{+2}$ sec$^{-1}$ um weniger als $\pm50$ %.

Die Vorteile dieses annähernd newtonschen Viskositätsverhaltens liegen u.a. darin, daß sich die Wirkstoffdispersionen, insbesondere nach der Aufkonzentrierung leichter Pumpen lassen, als dies bei strukturviskosen Dispersionen der Fall ist. Beim Sprühtrocknen haben die annähernd newtonschen Wirkstoffdispersionen außerdem den Vorteil, daß sich die Parameter des Sprühkopfes leichter optimieren lassen und daß sich diese Dispersionen im Sprühkopf unkritischer verhalten.

Im Vergleich zu teilabgebauten Sojaproteinen mit geringen Abbaugraden < 5 %, lassen sich mit den erfindungsgemäß verwendeten Sojaproteinen Xanthophyll-haltige Trockenpulver mit verbesserter Farbstärke sowie verbesserter Kaltwasser-Redispergierbarkeit herstellen.

Teilabgebaute Sojaproteine mit einem Abbaugrad größer 5 % zeigen überraschenderweise eine bessere Verträglichkeit mit den eingangs genannten wassermischbaren Lösungsmitteln. Dadurch sind konzentriertere Fahrweisen und somit ein wirtschaftlicheres Verfahren zur Herstellung der erfindungsgemäßen Trockenpulver möglich.

Ferner konnte beobachtet werden, daß mit dem erfindungsgemäßen Verfahren die Zusammenlagerung der Xanthophylle zu H-Aggregaten vermieden wird.

Die Aggregation von Carotinoiden ist ein in der Literatur bereits bekanntes und in zahlreichen Publikationen beschriebenes Phänomen [P. Song, T. A. Moore, Photochemistry and Photobiology, 19, 435-441 (1974); A. V. Ruban, P. Horton, A. J. Young, J. Photochem. Photobiol. B: Biol., 21, 229-234 (1993); V. R. Salares, N.M. Young, P. R. Carey, H. J. Bernstein, Journal of Raman Spectroscopy, 6(6), 282-288 (1977)].

Carotinoid-Aggregate können beispielsweise dadurch erzeugt werden, daß man eine Lösung eines Carotinoids in einem wassermischbaren, organischen Lösungsmittel wie z.B. Isopropanol, Ethanol, Aceton oder Tetrahydrofuran mit Wasser vermischt.

So können, wie in der oben genannten Literatur beschrieben, bei Wahl der richtigen Mengenverhältnisse von Wasser und organischem Lösungsmittel entweder sogenannte H- oder J-Aggregate erzeugt werden.

Unter H-Aggregaten versteht man eine "kartenspielähnliche" Stapelung der Polyenketten (card-stack aggregate), die sich im UV/Vis-Spektrum durch das Auftreten einer neuen, im Vergleich zur Absorption der monomer vorliegenden Formen hypsochrom verschobenen Bande im Bereich zwischen 320 und 400 nm charakterisieren läßt. J-Aggregate dagegen stellen entweder eine lineare Kopf-Schwanz Verknüpfung (head-tail aggregates) der Polyene dar oder sie sind fischgrätenartig angeordnet (herringbone aggregates). Beide Anordnungen bewirken eine bathochrome Verschiebung der UV-Absorption der Polyene.

Fütterungsversuche an Forellen haben gezeigt, daß H-Aggregate von Xanthophyllen, insbesondere die H-Aggregate von Astaxanthin eine schlechtere Bioverfügbarkeit aufweisen als die entsprechenden J-Aggregate, was sich als ein weiterer Vorteil der nach dem erfindungsgemäßen Verfahren hergestellten Trockenpulver darstellt.

Die oben genannten Trockenpulver eignen sich insbesondere als Zusatz zu Lebens- und Tierfuttermitteln sowie als Zusatz zu pharmazeutischen Zubereitungen. Typische Einsatzgebiete für die Carotinoid-haltigen Trockenpulver im Tierfuttermittelbereich sind beispielsweise die Fischpigmentierung in der Aquakultur sowie die Eidotter- und Broilerhautpigmentierung in der Geflügelzucht.

In den nachfolgenden Beispielen wird die Durchführung des erfindungsgemäßen Verfahrens näher erläutert.

Beispiel 1

Herstellung eines Sojaprotein-Hydrolysats durch enzymatischen Abbau

In einem 2 l Becherglas wurden 600 ml Wasser und 40 g Sojaprotein-Isolat (Proteingehalt 85 Gew.-%) vorgelegt und unter Rühren auf 60°C erwärmt. Dann wurde der pH-Wert mit 1 M Natronlauge auf 9,0 eingestellt, 0,5 g Bromelain zugegeben und 45 Minuten lang bei 60°C weitergerührt. Anschließend wurde der pH-Wert erneut mit 1 M Natronlauge auf 9,0 eingestellt. Anhand des Natronlauge-Verbrauchs berechnete sich der DH-Wert von 7%. Das Enzym wurde anschließend durch 2-minütiges Erhitzen der Lösung auf 100°C desaktiviert.

Beispiel 2

Astaxanthin Trockenpulver

In einer beheizbaren Vorlage wurden bei einer Temperatur von 30°C 48 g kristallines Astaxanthin und 20 g α-Tocopherol in 350 g eines azeotropen Isopropanol/Wasser-Gemischs bei Raumtemperatur suspendiert. Die Wirkstoffsuspension wurde dann auf 100°C erwärmt und bei einer Flussrate von 2,1 kg/h kontinuierlich mit weiterem Isopropanol/Wasser-Azeotrop der Temperatur 226°C und einer Flussrate von 2,6 kg/h vermischt, wobei sich Astaxanthin bei einer sich einstellenden Mischungstemperatur von 172°C bei einem Druck von 60 bar auflöste. Diese Wirkstofflösung wurde unmittelbar anschließend mit einer wässrigen Phase, bestehend aus einer Lösung von 72 g teilabgebautem Sojaprotein mit einem Abbaugrad von 7 %, 200 g Glucosesirup in 6000 g destilliertem Wasser, in dem der pH-Wert mit 1 M NaOH auf pH 9,5 eingestellt wurde, bei einer Flussrate von 35,8 kg/h vermischt.

Die bei der Mischung entstandenen Wirkstoffteilchen wiesen im Isopropanol/Wasser-Gemisch eine Teilchengröße von 174 nm auf, bei einem E1/1-Wert von 119.

Anschließend wurde die Wirkstoff-Suspension am Dünnfilmverdampfer auf eine Konzentration von ca. 3,4 % Wirkstoffgehalt aufkonzentriert und sprühgetrocknet. Das Trockenpulver wies einen Astaxanthin-Gehalt von 12,3 Gew.-% auf. Das in Wasser redispergierte Trockenpulver hatte eine Teilchengröße von 200 nm und wies einen E1/1-Wert von 101 auf.

Beispiel 3

Astaxanthin Trockenpulver

Zunächst wurden 48 g kristallines Astaxanthin, 1,6 g Ascorbylpalmitat und 20 g $\alpha$-Tocopherol in 350 g eines azeotropen Isopropanol/Wasser-Gemischs bei Raumtemperatur suspendiert. Diese Wirkstoffsuspension wurde dann auf 88°C erwärmt und bei einer Flussrate von 2,1 kg/h kontinuierlich mit weiterem heißem Isopropanol/Wasser-Azeotrop bei einer Flussrate von 2,7 kg/h vermischt, wobei sich Astaxanthin bei einer sich einstellenden Mischungstemperatur von 165°C bei einem Druck von 60 bar auflöste. Die Wirkstofflösung wurde sodann mit einer wässrigen Phase, bestehend aus einer Lösung von 103 g teilabgebautem Sojaprotein, hergestellt gemäß Beispiel 1 und 163 g Glucose in 10800 g destilliertem Wasser, in dem der pH-Wert mit 1 M NaOH auf pH 9,5 eingestellt worden war, bei einer Flussrate von 60 kg/h vermischt.

Die bei der Mischung entstandenen Wirkstoffteilchen wiesen im Isopropanol/Wasser-Gemisch eine Teilchengröße von 153 nm auf, bei einem E1/1-Wert von 124. Anschließend wurde diese Wirkstoff-Dispersion am Dünnfilmverdampfer auf eine Konzentration von ca. 3,6 % Wirkstoffgehalt aufkonzentriert und sprühgetrocknet. Das Trockenpulver wies einen Astaxanthin-Gehalt von 13,0 Gew.-% auf. Das in Wasser redispergierte Trockenpulver hatte eine mittlere Teilchengröße von 400 nm und wies einen E1/1-Wert von 106 auf.

Beispiel 4

Canthaxanthin Trockenpulver

Zunächst wurden 48 g kristallines Canthaxanthin, 4 g Ascorbylpalmitat und 16 g $\alpha$-Tocopherol in 350 g eines azeotropen Isopropanol/Wasser-Gemischs bei Raumtemperatur suspendiert. Diese Wirkstoffsuspension wurde dann auf 88°C erwärmt und bei einer Flussrate von 2,9 kg/h kontinuierlich mit weiterem heißem Isopropanol/ Wasser-Azeotrop bei einer Flussrate von 4,8 kg/h vermischt, wobei sich Canthaxanthin bei einer sich einstellenden Mischungstemperatur von 175 °C und bei einem Druck von 60 bar auflöste. Diese Wirkstofflösung wurde sodann mit einer wässrigen Phase, bestehend aus einer Lösung von 120 g teilabgebautem Sojaprotein mit einem Abbaugrad von 7 % und 197 g Glucose in 7400 g destilliertem Wasser, in dem der pH-Wert mit 1 M NaOH auf pH 9,5 eingestellt worden war, bei einer Flussrate von 52 kg/h vermischt.

Die bei der Mischung entstandenen Wirkstoffteilchen wiesen im Isopropanol/Wasser-Gemisch eine Teilchengröße von 139 nm auf, bei einem E1/1-Wert von 134. Anschließend wurde diese Wirkstoff-Dispersion am Dünnfilmverdampfer auf eine Konzentration von ca. 3,3 % Wirkstoffgehalt aufkonzentriert und sprühgetrocknet. Das Trockenpulver wies einen Canthaxanthin-Gehalt von 12,5 Gew.-% auf. Das in Wasser redispergierte Trockenpulver hatte eine mittlere Teilchengröße von 224 nm und wies einen E1/1-Wert von 125 auf.

Beispiel 5

Citranaxanthin Tockenpulver

100 g kristallines Citranaxanthin, 59 g teilabgebaute Sojaprotein mit einem Abbaugrad von 7 % und 50 g Natriumascorbat wurden in 280 g VE-Wasser bei Raumtemperatur suspendiert. Die Wirkstoff-Suspension wurde dann zusammen mit ca. 400 g Zirkonoxid-Keramikmahlperlen vom Durchmesser 1 mm in einer 1000 ml-Glasflasche auf einer Hochleistungsdispergiermaschine (Red-Devil®, Fa. Erichsen, Deutschland) dispergiert. Nach einer Mahldauer von 1 Stunde wurde die gemahlene Suspension unter Stickstoff zu einer wäßrigen Lösung von 130 g eines teilabgebauten Sojaproteins mit einem Abbaugrad von 7 %, 200 g Saccharose in Gegenwart von 1 g Ascorbylpalmitat und 1,5 g $\alpha$-Tocopherol gegeben. Nach Homogenisierung der Mischung wurde die Suspension anschließend mittels Sprühkühlung getrocknet. Man erhielt ein Citranaxanthin Trockenpulver mit einem Wirkstoffgehalt von 13 Gew.-%.

**Patentansprüche**

1. Verfahren zur Herstellung von Trockenpulvern eines oder mehrerer Sauerstoff-haltiger Carotinoide durch

    a) Dispergieren eines oder mehrerer Sauerstoff-haltiger Carotinoide in einer wäßrigen molekulardispersen oder kolloiddispersen Lösung eines Schutzkolloids und

    b) Überführung der gebildeten Dispersion in ein Trockenpulver durch Abtrennung des Wassers und gegebenenfalls zusätzlich verwendeter Lösungsmittel und Trocknung, gegebenenfalls in Gegenwart eines Überzugsmaterials,

**dadurch gekennzeichnet, daß** man als Schutzkolloid im Verfahrensschritt a) mindestens ein teilabgebautes Sojaprotein mit einem Abbaugrad von größer 5 % verwendet.

2. Verfahren nach Anspruch 1, **dadurch gekennzeichnet, daß** es sich beim Dispergierschritt a) um die Herstellung einer Suspension eines oder mehrerer Sauerstoff-haltiger Carotinoide in einer wäßrigen molekulardispersen oder kolloiddispersen Lösung eines teilabgebauten Sojaproteins mit einem Abbaugrad von größer 5 % handelt.

3. Verfahren nach Anspruch 2, **dadurch gekennzeichnet, daß** man die im Verfahrensschritt a) hergestellte Suspension vor der Überführung in ein Trockenpulver mahlt.

4. Verfahren nach Anspruch 1, **dadurch gekennzeichnet, daß** das Dispergieren in der Stufe a) folgende Schritte enthält:

a$_1$) Lösen eines oder mehrerer Sauerstoff-haltiger Carotinoide in einem mit Wasser mischbaren, organischen Lösungsmittel oder in einer Mischung aus Wasser und einem mit Wasser mischbaren, organischen Lösungsmittel oder

a$_2$) Lösen eines oder mehrerer Sauerstoff-haltiger Carotinoide in einem mit Wasser nicht mischbaren, organischen Lösungsmittel und

a$_3$) Mischen der nach a$_1$) oder a$_2$) erhaltenen Lösung mit einer wäßrigen molekulardispersen oder kolloiddispersen Lösung eines teilabgebauten Sojaproteins mit einem Abbaugrad von größer 5 %, wobei die hydrophobe Phase des Carotinoids als nanodisperse Phase entsteht.

5. Verfahren nach einem der Ansprüche 1 bis 4, **dadurch gekennzeichnet, daß** man als Schutzkolloid mindestens ein teilabgebautes Sojaprotein mit einem Abbaugrad von 6 bis 12 % verwendet.

6. Verfahren nach einem der Ansprüche 1 bis 5, **dadurch gekennzeichnet, daß** es sich bei den Sauerstoff-haltigen Carotinoiden um Verbindungen, ausgewählt aus der Gruppe, bestehend aus Astaxanthin, Canthaxanthin, Lutein, Zeaxanthin, Citranaxanthin und β-Apo-8'-Carotinsäureethylester handelt.

7. Verfahren nach Anspruch 6, **dadurch gekennzeichnet, daß** man

a) Astaxanthin und/oder Canthaxanthin in einem mit Wasser mischbaren, organischen Lösungsmittel oder einer Mischung aus Wasser und einem mit Wasser mischbaren, organischen Lösungsmittel bei Temperaturen größer 30°C löst,

b) die erhaltene Lösung mit einer wäßrigen molekulardispersen oder kolloiddispersen Lösung eines teilabgebauten Sojaproteins mit einem Abbaugrad von 6 bis 9 % mischt und

c) die gebildete Dispersion in ein Trockenpulver überführt.

8. Verfahren nach Anspruch 7, **dadurch gekennzeichnet, daß** man als Carotinoid Astaxanthin verwendet.

9. Trockenpulver von Sauerstoff-haltigen Carotinoiden, erhältlich nach einem Verfahren, definiert gemäß einem der Ansprüche 1 bis 8.

10. Trockenpulver nach Anspruch 9, enthaltend Sauerstoff-haltige Carotinoide, ausgewählt aus der Gruppe, bestehend aus Astaxanthin, Canthaxanthin, Lutein, Zeaxanthin, Citranaxanthin und β-Apo-8'-Carotinsäureethylester.

11. Trockenpulver nach einem der Ansprüche 9 oder 10 mit einem Carotinoidgehalt von 0,1 bis 30 Gew.-%.

12. Trockenpulver nach einem der Ansprüche 9 bis 11, enthaltend 5 bis 20 Gew.-% Astaxanthin.

13. Trockenpulver nach einem der Ansprüche 9 bis 11, enthaltend 5 bis 20 Gew.-% Canthaxanthin.

**14.** Verwendung der Trockenpulver, definiert gemäß einem der Ansprüche 9 bis 13, als Zusatz zu Lebensmitteln, Pharmazeutika und/oder Tierfuttermitteln.

**Claims**

**1.** A process for preparing dry powders of one or more oxygenated carotenoids by

a) dispersing one or more oxygenated carotenoids in an aqueous molecular dispersion or colloidal dispersion of a protecting colloid and

b) converting the dispersion formed into a dry powder by removing the water and any solvents additionally used and drying, in the presence or absence of a coating material,

which comprises using as protecting colloid in process step a) at least one partially hydrolyzed soybean protein having a degree of hydrolysis greater than 5%.

**2.** A process as claimed in claim 1, wherein the dispersion step a) is the production of a suspension of one or more oxygenated carotenoids in an aqueous molecular dispersion or colloidal dispersion of a partially hydrolyzed soybean protein having a degree of hydrolysis greater than 5%.

**3.** A process as claimed in claim 2, wherein the suspension produced in process step a) is ground before conversion to a dry powder.

**4.** A process as claimed in claim 1, wherein the dispersion in stage a) comprises the following steps:

$a_1$) dissolving one or more oxygenated carotenoids in a water-miscible organic solvent or in a mixture of water and a water-miscible organic solvent or

$a_2$) dissolving one or more oxygenated carotenoids in a water-immiscible organic solvent and

$a_3$) mixing the solution obtained after $a_1$) or $a_2$) with an aqueous molecular dispersion or colloidal dispersion of a partially hydrolyzed soybean protein having a degree of hydrolysis greater than 5%, the hydrophobic phase of the carotenoid being produced as nanodisperse phase.

**5.** The process as claimed in one of claims 1 to 4, wherein the protecting colloid is at least one partially hydrolyzed soybean protein having a degree of hydrolysis of from 6 to 12%.

**6.** A process as claimed in one of claims 1 to 5, wherein the oxygenated carotenoids are compounds selected from the group consisting of astaxanthin, canthaxanthin, lutein, zeaxanthin, citranaxanthin and β—apo—8'—arotinic acid ethyl ester.

**7.** A process as claimed in claim 6 wherein

a) astaxanthin and/or canthaxanthin is dissolved in a water-miscible organic solvent or a mixture of water and a water-miscible organic solvent at temperatures above 30°C,

b) the resultant solution is mixed with an aqueous molecular dispersion or colloidal dispersion of a partially hydrolyzed soybean protein having a degree of hydrolysis of from 6 to 9% and

c) the dispersion formed is converted into a dry powder.

**8.** A process as claimed in claim 7, wherein the carotenoid is astaxanthin.

**9.** A dry powder of oxygenated carotenoids obtainable by a process defined according to one of claims 1 to 8.

**10.** A dry powder as claimed in claim 9 comprising oxygenated carotenoids selected from the group consisting of astaxanthin, canthaxanthin, lutein, zeaxanthin, citranaxanthin and β—apo—8'—carotinic acid ethyl ester.

**11.** A dry powder as claimed in one of claims 9 or 10 having a carotenoid content of from 0.1 to 30% by weight.

**12.** A dry powder as claimed in one of claims 9 to 11, comprising from 5 to 20% by weight of astaxanthin.

**13.** A dry powder as claimed in one of claims 9 to 11, comprising from 5 to 20% by weight of canthaxanthin.

**14.** The use of the dry powder defined according to one of claims 9 to 13 as additive to foods, pharmaceuticals and/or animal feeds.

**Revendications**

**1.** Procédé pour la préparation de poudres sèches d'un ou plusieurs caroténoïdes contenant de l'oxygène par

   a) dispersion d'un ou plusieurs caroténoïdes contenant de l'oxygène dans une solution aqueuse d'un colloïde protecteur à l'état de dispersion moléculaire ou de dispersion colloïdale et
   b) conversion de la dispersion obtenue en une poudre sèche par séparation de l'eau et du solvant éventuellement utilisé, et séchage, le cas échéant en présence d'une matière de revêtement,

   **caractérisé par le fait que** l'on utilise en tant que colloïde protecteur au stade opératoire a) au moins une protéine de soja partiellement dégradée, à un taux de dégradation supérieur à 5%.

**2.** Procédé selon la revendication 1, **caractérisé par le fait que** l'opération de dispersion a) consiste en la préparation d'une suspension d'un ou plusieurs caroténoïdes contenant de l'oxygène dans une solution aqueuse, à l'état de dispersion moléculaire ou de dispersion colloïdale, d'une protéine de soja partiellement dégradée, à un taux de dégradation supérieur à 5%.

**3.** Procédé selon la revendication 2, **caractérisé par le fait que** la suspension préparée au stade opératoire a) est broyée avant la conversion en une poudre sèche.

**4.** Procédé selon la revendication 1, **caractérisé par le fait que** la dispersion du stade opératoire a) comprend les étapes suivantes :

   a$_1$) dissolution d'un ou plusieurs caroténoïdes contenant de l'oxygène dans un solvant organique miscible à l'eau ou dans un mélange d'eau et d'un solvant organique miscible à l'eau, ou bien
   a$_2$) dissolution d'un ou plusieurs caroténoïdes contenant de l'oxygène dans un solvant organique non miscible à l'eau et
   a$_3$) mélange de la solution obtenue en a$_1$) ou a$_2$) avec une solution aqueuse, à l'état de dispersion moléculaire ou de dispersion colloïdale, d'une protéine de soja partiellement dégradée, à un taux de dégradation supérieur à 5%, la phase hydrophobe du caroténoïde constituant la phase nanodispersée.

**5.** Procédé selon l'une des revendications 1 à 4, **caractérisé par le fait que** l'on utilise en tant que colloïde protecteur au moins une protéine de soja partiellement dégradée à un taux de dégradation de 6 à 12%.

**6.** Procédé selon l'une des revendications 1 à 5, **caractérisé par le fait que** les caroténoïdes contenant de l'oxygène sont des composés choisis dans le groupe consistant en l'astaxanthine, la canthaxanthine, la lutéine, la zéaxanthine, la citranaxanthine et le β-apo-8'-caroténate d'éthyle.

**7.** Procédé selon la revendication 6, **caractérisé par le fait que**

   a) on dissout l'astaxanthine et/ou la canthaxanthine dans un solvant organique miscible à l'eau ou dans un mélange d'eau et d'un solvant organique miscible à l'eau à des températures supérieures à 30°C,
   b) on mélange la solution obtenue avec une solution aqueuse, à l'état de dispersion moléculaire ou de dispersion colloïdale, d'une protéine de soja partiellement dégradée, à un taux de dégradation de 6 à 9% et
   c) on convertit la dispersion formée en une poudre sèche.

**8.** Procédé selon la revendication 7, **caractérisé par le fait que** le caroténoïde utilisé est l'astaxanthine.

9. Poudre sèche de caroténoïdes contenant de l'oxygène, obtenue par un procédé tel que défmi dans l'une des revendications 1 à 8.

10. Poudre sèche selon la revendication 9, contenant des caroténoïdes qui contiennent eux-mêmes de l'oxygène et choisis dans le groupe consistant en l'astaxanthine, la canthaxanthine, la lutéine, la zéaxanthine, la citranaxanthine et le β-apo-8'-caraténate d'éthyle.

11. Poudre sèche selon l'une des revendications 9 ou 10, à une teneur en caroténoïdes de 0,1 à 30% en poids.

12. Poudre sèche selon l'une des revendications 9 à 11, contenant 5 à 20% en poids d'astaxanthine.

13. Poudre sèche selon l'une des revendications 9 à 11, contenant 5 à 20% en poids de canthaxanthine.

14. Utilisation de la poudre sèche définie dans l'une des revendications 9 à 13, en tant qu'additif à des produits alimentaires, des produits pharmaceutiques et/ou des aliments pour le bétail.